# EUROPÄISCHE PATENTSCHRIFT

(11) **EP 2 864 779 B1**
(45) Veröffentlichungstag und Bekanntmachung des Hinweises auf die Patenterteilung: **27.06.2018**
(21) Anmeldenummer: 13753128.1
(22) Anmeldetag: 14.08.2013
(51) Int. Cl.: G01N 33/18

(54) **VERFAHREN UND VORRICHTUNG ZUR MESSUNG DES GASGEHALTS IN EINER FLÜSSIGKEIT SOWIE VERWENDUNG EINER SOLCHEN VORRICHTUNG**
METHOD AND DEVICE FOR MEASURING THE GAS CONTENT OF A LIQUID AND USE OF SUCH A DEVICE
PROCÉDÉ ET DISPOSITIF DE MESURE DE LA TENEUR EN GAZ DANS UN LIQUIDE AINSI QU'UTILISATION D'UN TEL DISPOSITIF

(30) Priorität: 21.08.2012 EP 12181119
(43) Veröffentlichungstag der Anmeldung: 29.04.2015
(73) Patentinhaber: Siemens Aktiengesellschaft, 80333 München (DE)
(72) Erfinder: MATTEJAT, Arno, 91056 Erlangen (DE)
(86) Internationale Anmeldenummer: PCT/EP2013/067000
(87) Internationale Veröffentlichungsnummer: WO 2014/029675

(56) Entgegenhaltungen:
- DE-A1- 4 400 385
- GB-A- 1 085 825
- US-A- 4 681 601
- US-A- 5 635 631
- US-A1- 2002 194 907

## Beschreibung

Die Erfindung betrifft ein Verfahren zur Überwachung und Messung des Gasgehalts in einer Flüssigkeit nach dem Oberbegriff des Anspruchs 1.

Die Erfindung betrifft weiterhin eine Vorrichtung zur Überwachung und Messung des Gasgehalts in einer Flüssigkeit nach dem Oberbegriff des Anspruchs 10 sowie eine Verwendung einer solchen Vorrichtung.

Ein derartiges Verfahren und eine derartige Vorrichtung gehen aus der DE 102 13 076 B4, Absatz [0004] als bekannt hervor.

In vielen technischen Bereichen ist eine Messung des Gasgehalts in Betriebsflüssigkeiten erforderlich, da das Vorliegen von Gas die Eigenschaften dieser Flüssigkeiten beeinflusst. Eine Messung des Gasgehalts ist daher z.B. für die Dimensionierung und Konstruktion von Maschinenteilen und zur Entwicklung von Flüssigkeiten mit günstigem Gasabscheideverhalten relevant. Insbesondere ist es wichtig, den Gasgehalt einer Schmierstoffflüssigkeit oder einer Heiz- bzw. Kühlflüssigkeit zu kennen.

Die Messung von Gas in Flüssigkeiten ist beispielsweise von Bedeutung für den störungsfreien Betrieb einer elektrochemischen Zelle, wie z.B. einer Brennstoffzelle. Im Betrieb einer Brennstoffzelle muss entstehende Verlustwärme aus dem aktiven Bereich der Brennstoffzelle weitgehend entfernt werden, um lokale Überhitzungen (sogenannte "hot spots") zu vermeiden. Dies erfolgt am effektivsten durch ein flüssiges Kühlmittel, das die Brennstoffzelle durchströmt.

In einem Brennstoffzellenstapel mit Brennstoffzellen basierend auf der Polymer-Elektrolyt-Membran (PEM)-Technologie befinden sich die Elektroden an der einer Elektrolyt-Membran bzw. Katalysatorschicht abgewandten Seite im Kontakt mit jeweils einer so genannten Bipolarplatte oder Kühleinheit. Die Bipolarplatte hat die Aufgabe, die einzelnen Brennstoffzellen (medienseitig) zu trennen, für Stromfluss im Zellenstapel zu sorgen und die Reaktionswärme zu entfernen.

Neben der Stromleitung und der Führung der Reaktanten Wasserstoff und Sauerstoff, erfüllen die Bipolarplatten ihre Kühlfunktion, indem ein Kühlmittel, insbesondere Wasser, durch diese geleitet wird. Wegen seiner hohen spezifischen Wärmekapazität, der geringen elektrischen Leitfähigkeit, der guten Medienverträglichkeit und der niedrigen Betriebskosten kommt bei PEM-Brennstoffzellen hauptsächlich deionisiertes Wasser (Deionat) als Kühlmittel zum Einsatz. Das Kühlmittel darf nur eine sehr geringe Leitfähigkeit haben und sollte möglichst gasfrei sein, damit sich keine Gasblasen an der Oberfläche der Bipolarplatten festsetzen, da Bereiche mit Gasblasen schlechter gekühlt werden können und daher lokal überhitzt werden. Aufgrund physikalischer und konstruktiver Vorgaben ist jedoch nicht auszuschließen, dass auch ursprünglich gasfreies Kühlwasser bei seinem Einsatz Gas aufnimmt und mit Blasen versehen ist.

In großen Heizungsanlagen werden sog. Vakuum-Entgaser eingesetzt, um gelöste Luft aus dem Heizungswasser zu entfernen. Ähnliche Einrichtungen können auch in Brennstoffzellenanlagen benutzt werden, um Gas aus dem Kühlwasser zu entfernen. Ziel dieser Methoden ist, kein Zwei-Phasen-Gemisch (Gas/Wasser) im Kühlwasser zuzulassen, so dass eine Gasansammlung und damit eine lokale Überhitzung an der Bipolarplatte nicht auftreten können.

Aus der eingangs erwähnten DE 102 13 076 B4 ist es bekannt, eine Probe einer Flüssigkeit, deren Gasgehalt zu messen ist, in einer Messkammer durch, z.B., kurzzeitige Druckentlastung zu entspannen. Der sich nach dem Entspannen einstellende Druck und die Probentemperatur werden dann gemessen. Daraus wird nach dem Henry'schen Gesetz der Gasgehalt berechnet.
Aus der DE 44 00 385 A1 ist es bekannt, den Gasgehalt einer durch eine Leitung fließenden Flüssigkeit kontinuierlich zu messen. Dazu wird die Flüssigkeit vor einer Drosselstelle verdichtet, um alle Gasanteile vollständig zur Lösung zu bringen, und hinter der Drosselstelle entspannt. Durch Messung der Trübung der Flüssigkeit vor und hinter der Drosselstelle wird die Gasentlösung detektiert. Anhand der detektierten Gasentlösung wird ein hinter der Drosselstelle liegendes Proportionalventil über einen Regler derart gesteuert, dass der Bestandteil des ungelösten Gases in der Regelstrecke zwischen Drosselstelle und Proportionalventil möglichst klein ist. Bei konstanter Temperatur wird der Druck der Flüssigkeit in der Regelstrecke gemessen und nach dem Henry'schen Gesetz (oder Henry-Dalton'schen Gesetz) der Gasgehalt der Flüssigkeit berechnet.

Der Erfindung liegt die Aufgabe zugrunde, eine schnelle und zuverlässige Kontrolle des Gasgehalts einer Flüssigkeit, z.B. eines Kühlmittels, zu ermöglichen.
Die Aufgabe wird erfindungsgemäß durch das in Anspruch 1 angegebene Verfahren gelöst, von dem vorteilhafte Weiterbildungen in den Unteransprüchen angegeben sind.
Die Aufgabe wird weiterhin erfindungsgemäß durch die in Anspruch 10 angegebene Vorrichtung gelöst.

Gegenstand der Erfindung ist schließlich auch eine Verwendung einer derartigen Vorrichtung zur Messung des Gasgehaltes in einem Kühlmittel einer elektrochemischen Zelle, insbesondere einer Brennstoffzelle.

Die in Bezug auf das Verfahren nachstehend aufgeführten Vorteile und bevorzugten Ausführungen lassen sich sinngemäß auf die Vorrichtung und ihre Verwendung übertragen.

Die Erfindung beruht auf der bekannten Erkenntnis, dass bei der Entspannung einer Flüssigkeit mit gelöstem Gas, das Gas in Form von kleinen Blasen ausfällt, die zu einer Trübung der Flüssigkeit führen. Unter Trübung wird hierbei eine Veränderung der Transmission und der Reflektion der Teilmenge für Wellen verstanden, die durch eine Veränderung der Zusammensetzung Flüssigkeit/Gas der Teilmenge hervorgerufen wird. Quantitativ wird diese Veränderung durch den Trübungswert ausgedruckt. Um bestimmen zu können, ob Gas enthalten ist, ist es somit zunächst erforderlich die Trübung der Flüssigkeit bei der Entspannung der Flüssigkeit in der Messzelle zu detektieren. Zudem sind Sensoren notwendig, mit deren Hilfe der Druck und die Temperatur in der Messzelle zum Zeitpunkt des Eintretens der Trübung bestimmt werden. Die erfassten Werte für Druck und Temperatur beim Eintreten der Trübung werden dabei mit bekannten Löslichkeitskennlinien verglichen, um die Menge an Gas in der Flüssigkeit unter den gemessenen Bedingungen zu bestimmen. Wenn es sich bei der Flüssigkeit z.B. um Wasser handelt, ist die Löslichkeit von Luft und anderen Gasen in Wasser in Abhängigkeit von Druck und Temperatur der Umgebung an sich bereits bekannt. Alternativ werden vorab Kenndaten für die Löslichkeit von dem in der Flüssigkeit aufgelösten Gas experimentell erfasst und hinterlegt, welche später beim der Messung des Gasgehalts in der entsprechende Flüssigkeit herangezogen werden.

Die Messung kann somit folgendermaßen kurz zusammengefasst werden: der Druck und die Temperatur in der Messzelle werden derart geregelt, dass eine detektierbare Trübung auftritt und beim Auftreten der Trübung die Menge an Gas durch die Löslichkeitskenndaten für das Gas bestimmt wird, wobei hierfür die Druck- und Temperaturwerte beim Auftreten der Trübung verwendet werden.

Es existieren umfangreiche Datensammlungen und Veröffentlichungen zum Thema Löslichkeit eines Gases im Wasser als Funktion des Druckes und Temperatur, die auf dem Henry-Gesetz zur Beschreibung des Löslichkeitsverhaltens von flüchtigen Substanzen in einer Flüssigkeit gestützt sind. Eine graphische Darstellung der Löslichkeit von Stickstoff in Wasser als Funktion des Druckes und der Temperatur ist z.B. aus Abb. B I-4.1 der Physiko-Chemische Datensammlung / R44 der Kraftwerk Union zu entnehmen. Eine tabellarische Zusammenfassung der Löslichkeit verschiedener Gase in Wasser in Abhängigkeit der Temperatur ist in MESSER GRIESHEIM Gase-Handbuch, Tafel 29 auf S. 92, Ausgabe 1969 veröffentlicht. Eine weitere graphische Darstellung der Löslichkeit von Luft in Wasser in Abhängigkeit von Druck und Temperatur findet sich im Artikel "Druckausdehnungsgefäße in Heizungsanlagen" von J. Spang in IKZ-Haustechnik, Ausgabe 4/1996, Seite 30 ff, Bild 2.

Die Temperatur- und Druckwerte, mit denen verglichen wird, stammen somit aus Quellen, wie sie für jedes Gas aus einschlägigen Werken und Tabellen entnommen werden können (Kennlinien für Löslichkeit eines Gases in Abhängigkeit von Temperatur und Druck). Aus solchen Quellen ist zu entnehmen, welche Gasmenge maximal bei einer bestimmten Temperatur und einem bestimmten Druck gelöst sein kann. Unterschreitet der Druck bei konstanter Temperatur den Wert für den Druck oder übersteigt die Temperatur bei konstantem Druck den Wert für die Temperatur, dann beginnt das Gas aus der Lösung als Blasen auszufallen und wird damit als Trübung sichtbar.

Vorzugsweise wird die Teilmenge mit Licht bestrahlt. Hierfür sind eine Lichtquelle und mindestens ein Lichtdetektor vorgesehen. Die Messezelle ist dabei durchlässig für das Licht der Lichtquelle.

Alternativ wird die Teilmenge bevorzugt akustisch mit Ultraschall bestrahlt. In diesem Fall sind ein Ultraschallsender und ein Ultraschalldetektor erforderlich.

Der ermittelte Trübungswert stellt ein Maß für die Veränderung der Zusammensetzung der Flüssigkeit mit sinkendem Druck dar. Der Trübungswert wird insbesondere durchgehend oder in Zeitabständen von wenigen Sekunden bis zu vielen Stunden bestimmt und mit einem Schwellwert verglichen. Der Schwellwert definiert dabei das Eintreten der Trübung in der Flüssigkeit. Wenn bei der Messung somit der Trübungswert den Schwellwert erreicht hat oder diesen überschreitet, ist eine Trübung der Flüssigkeit detektiert, die qualitativ auf das Vorliegen von Gas in der Flüssigkeit deutet.

Zur Bestimmung des Gasgehalts werden die Druck- und die Temperaturmessung in der Messzelle beim Eintreten der Trübung berücksichtigt. Aufgrund von vorhandenen Kenndaten kann aus der Temperatur und dem Druck, bei denen die Trübung einsetzt, auf die Menge des gelösten Gases geschlossen werden. Beispielsweise deutet ein frühes Eintreten der Trübung bei einem Druck, der knapp unter dem Umgebungsdruck ist, auf eine große Menge an Gas.

Wenn bei der Messung Gas festgestellt wird, wird insbesondere ein entsprechendes Signal ausgegeben, das an eine Steuereinheit oder an eine optische Anzeige weitergeleitet wird. Mit diesem Signal wird insbesondere ein Vakuum-Entgaser eingeschaltet und solange betrieben, bis die Flüssigkeit wieder gasfrei ist.

Beim Einsatz der Vorrichtung zur Messung eines Gasgehalts in einer elektrochemischen Zelle wie z.B. einer Brennstoffzelle kann die Messzelle dabei an einer Neben- oder Bypassleitung parallel zu einer Hauptleitung für den Kühlmittelkreislauf angeordnet sein, so dass lediglich ein kleiner Teil des Kühlmittels durch die Messzelle geleitet wird. Alternativ kann die Messzelle in der Hauptleitung integriert sein, so dass ein Umleiten des Kühlmittels zu Messzwecken nicht erforderlich ist.
Die Überwachung des Gasgehalts in der Flüssigkeit kann diskontinuierlich erfolgen, indem aus dem Hauptstrom in der Hauptleitung eine kleine Teilmenge durch eine Pumpe in die Messzelle gefördert wird und nach dem Schließen eines Einlassventils die Pumpe so gesteuert wird, dass der Druck in der Messzelle langsam abfällt und die optische Messung durchgeführt wird.
Alternativ wird die Flüssigkeit jedoch kontinuierlich in die Messzelle zur Messung des Trübungswerts eingeleitet. Hierfür ist bevorzugt ein Regelventil zum kontinuierlichen Zufluss von Kühlmittel in die Messzelle vorgesehen. Zur Druckabsenkung in der Messzelle wird das Regelventil derart angesteuert, dass die gewünschten, temperaturabhängigen Druckwerte, die für die Messung benötigt werden, bei konstanter Pumpendrehzahl eingestellt werden. Der Druck in der Messzelle sinkt langsam, bis sich eine Trübung einstellt. Aus der zu diesem Zeitpunkt gemessenen Druck und Temperatur lässt sich die Gasbeladung ermitteln. Grenze zur Durchführung des erfindungsgemäßen Verfahrens ist ein Druck kurz oberhalb des Siededrucks (ca. 50 mbar oberhalb des Siededrucks). Oberhalb dieser Druckgrenze kann durch das Auftreten der Trübung festgestellt werden, ob in der Flüssigkeit Gas enthalten ist.
Eine weitere Alternative stellen ein festes Drosselventil und ein Absenken des Drucks durch die Pumpe dar. Die Auswertung erfolgt wie im obigen Absatz beschrieben.
Ein Vorteil der oben beschriebenen Messung ist, dass mit kleinen Volumina bzw. Teilmengen an Flüssigkeit und minimalen Mengen an ausfallendem Gas gearbeitet wird, so dass die Messung durch eine hohe Empfindlichkeit gekennzeichnet ist. Ein weiterer Vorteil ist, dass sie voll automatisch und ohne den Eingriff eines Bedieners ablaufen kann. Wenn der Gasgehalt eines Kühlmittels für eine elektrochemische Zelle gemessen wird, wird zudem die geprüfte Teilmenge nicht entsorgt, sondern sie wird nach der Messung der Trübung insbesondere in den Kühlmittelkreislauf der elektrochemischen Zelle zurück gepumpt.

Gemäß der Erfindung werden der Druck und/oder die Temperatur in der Messzelle derart angesteuert, dass sie oberhalb des Siedepunkts bleiben. Der Siedepunkt ist hierbei als ein Punkt an der Phasengrenze flüssig/gasförmig im Phasendiagramm (Dampfdruckkurve) der Flüssigkeit definiert, in dem die Sättigungstemperatur bzw. Siedetemperatur und der Sättigungsdampfdruck bzw. Siededruck erreicht ist. Insbesondere wird der Druck in der Messzelle derart angesteuert, dass er während der Messung bei der entsprechenden Temperatur etwa 50 mbar oberhalb des Siededrucks bleibt. Beim Herabsetzen des Drucks in der Messzelle verdampft die Flüssigkeit in Form kleiner Blasen, wenn bei der entsprechenden Temperatur der Siededruck erreicht ist. Derartige Dampfblasen haben den gleichen Einfluss auf die Trübung wie ein in der Flüssigkeit gelöstes Gas. Um eine Verfälschung der Messergebnisse für den Trübungswert aufgrund von Siedeblasen zu vermeiden, werden die Temperatur- und Drucksensoren benötigt, durch deren Signale unterschieden werden kann, ob es sich bei der Trübung um einen Effekt aus der Ausgasung oder aus der Verdampfung der Flüssigkeit handelt. Daher ist es vorgesehen, dass während der Durchführung des Verfahrens der Druck und die Temperatur insbesondere kontinuierlich oder in kurzen Zeitabständen gemessen werden, um rechtzeitig auf eine Annäherung an den Siedepunkt reagieren zu können.
Gemäß einer bevorzugten Variante wird der Schwellwert als Zahlenwert vorgegeben. Der Schwellwert kann dabei ein in der Analyseeinheit hinterlegter Wert sein, kann jedoch auch vor jeder Messung neu eingegeben werden.
Um Veränderungen der Flüssigkeit zu erkennen, welche nicht durch den Gasgehalt in der Teilmenge hervorgerufen sind, welche jedoch die Transmission- und Reflektionseigenschaften der Flüssigkeit beeinflussen, z.B. Verfärbungen oder Verschmutzungen oder Veränderungen an Messkomponenten (beispielsweise durch eine Schwächung der Lichtquelle), wird zweckdienlicherweise eine Lichtmessung zur Festlegung eines Referenzwertes durchgeführt bevor der Unterdruck eingestellt wird (d.h. noch bei Systemdruck). Der Referenzwert wird zum Beginn, bei noch nicht abgesenktem Druck in der Messzelle insbesondere über eine Licht- oder Ultraschallmessung gewonnen und definiert einen Anfangszustand der Flüssigkeit im Hinblick auf ihre Trübung. Eine Veränderung des Trübungswerts gegenüber dem Referenzwert würde somit auf das Vorliegen von Gas in der Flüssigkeit hindeuten.

Vorzugsweise wird der Referenzwert, sobald er ermittelt ist, mit einem Grenzwert verglichen. Aus diesem Vergleich wird eine Selbstüberwachung der Vorrichtung abgeleitet, mit deren Hilfe sich Verschmutzungen oder Geräteausfälle erkennen lassen. Wenn bei Umgebungsdruck eine Trübung der Flüssigkeit durch den Vergleich des Referenzwerts mit dem Grenzwert festgestellt wird, deutet dies insbesondere auf eine deutliche Verschmutzung der Messvorrichtung. Bei einem vordefinierten Schwellwert kann der Grenzwert durch den Schwellwert gebildet sein oder eine Funktion des Schwellenwerts sein (z.B. der Grenzwert entspricht 80 % des Schwellwerts). Im Normalfall liegt der Referenzwert unterhalb des Grenzwerts, d.h. die Flüssigkeit ist bei Umgebungs- bzw. Systemdruck "klar".

Alternativ zum vorgegebenen Zahlenwert für den Schwellwert wird der Schwellwert gemäß einer weiteren bevorzugten Variante auf Grundlage des gemessenen Referenzwertes berechnet. Um den Schwellwert zu bilden, wird hierbei insbesondere zum Referenzwert ein Toleranzbereich Δ addiert, durch welches eine Erhöhung der Trübung quantitativ ausgedrückt ist. Wenn der Trübungswert den Referenzwert um den Toleranzbereich Δ übersteigt (d.h. wenn der Trübungswert den Schwellwert erreicht und größer als der Schwellwert ist), liegt eine Trübung der Flüssigkeit vor.

Nach einer bevorzugten Variante wird ein durch die Flüssigkeit transmittierter Anteil des Lichts oder Ultraschalls gemessen, indem der Detektor derart angeordnet ist, dass die Messzelle sich zwischen der Lichtquelle oder dem Ultraschallsender und dem Detektor befindet. Nach einer alternativen Variante wird ein von der Flüssigkeit reflektierter Anteil des Lichts oder Ultraschalls gemessen, indem der Detektor an der Seite der Messzelle angeordnet ist, an der sich auch die Lichtquelle oder der Ultraschallsender befindet. Möglich sind auch Messsysteme, bei denen sowohl das transmittierte als auch das reflektierte Licht gemessen werden. Dabei wird berücksichtigt, dass Gasblasen innerhalb der Flüssigkeit die Intensität des durchgehenden Lichtes abschwächen und somit den Anteil des gestreuten Lichts erhöhen, so dass der Trübungswert steigt. Eine Alterung der Lichtquelle sowie eine Verschmutzung der lichtdurchlässigen Messzelle schwächt die Intensität des durchgehenden und des gestreuten Lichts, d.h. der Trübungswert bleibt konstant.

Im Hinblick auf eine störungsfreie Messung wird eine Geschwindigkeit der Flüssigkeit derart geregelt, dass Gasblasen von einer Wand der Messzelle ausgeschwemmt werden. Dadurch wird eine ungünstige Beeinflussung der Messergebnisse, insbesondere bei einem kontinuierlichen Durchlauf von Teilmengen der Flüssigkeit durch die Messzelle, vermieden.

Ein Ausführungsbeispiel der Erfindung wird anhand einer Zeichnung näher erläutert. Hierin zeigen:
- FIG 1: eine Vorrichtung zur diskontinuierlichen Überwachung des Gasgehalts eines Kühlmittels einer Brennstoffzelle,
- FIG 2: eine Vorrichtung zur kontinuierlichen Überwachung des Gasgehalts eines Kühlmittels einer Brennstoffzelle, und
- FIG 3: eine dritte Ausführungsvariante einer Vorrichtung zum Messen des Gasanteils in einer Flüssigkeit, und
- FIG 4: eine Anordnung einer Messzelle.

Gleiche Bezugszeichen haben in den verschiedenen Figuren die gleiche Bedeutung.

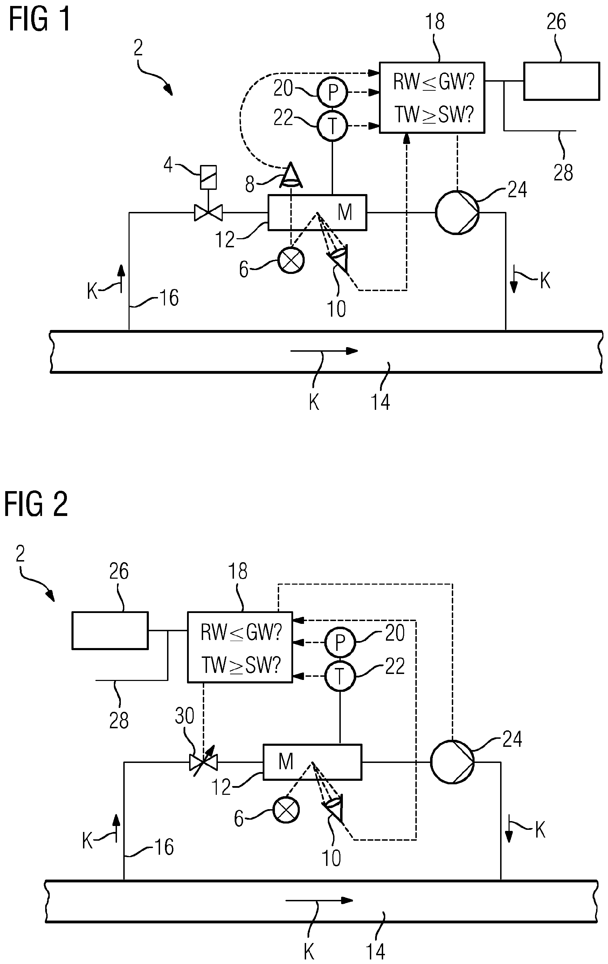

In FIG 1 ist eine erste Vorrichtung 2 zur Messung des Gasgehalts eines flüssigen Kühlmittels K, hier ein Kühlwasser für eine nicht näher gezeigte Brennstoffzelle, gezeigt. Die Vorrichtung 2 umfasst ein Einlassventil 4, eine Lichtquelle 6, zwei Detektoren 8, 10, die symbolisch als offene Augen dargestellt sind, sowie eine für das Licht der Lichtquelle 6 durchlässige Messzelle 12. Als Lichtquelle 6 eigenen sich LED, Glühlampen, Gasentladungslampen, etc.. Jeder der Lichtdetektoren kann z.B. Fototransistoren, Fotodioden oder Fotowiderstände umfassen.

Alternativ zur Lichtmessung kann anstelle der Lichtquelle 6 ein hier nicht näher gezeigter Ultraschallsender vorgesehen werden; entsprechend ist dann der Detektor 8, 10 ein Ultraschalldetektor.

Im gezeigten Ausführungsbeispiel wird mit Hilfe der Lichtdetektoren 8, 10 Licht aus der Lichtquelle 6 nach dem Kontakt mit dem Kühlwasser K in der Messzelle 12 gemessen. Ein erster Lichtdetektor 8 ist dabei für eine Messung des durch das Kühlwasser K in der Messzelle 12 transmittierten Lichts vorgesehen und ist dabei derart angeordnet, dass die Messzelle 12 sich zwischen ihm und der Lichtquelle 6 befindet. Ein zweiter Lichtdetektor 10 ist an der gleichen Seite der Messzelle 12 wie die Lichtquelle 6 angeordnet und misst dabei das von einer Teilmenge M am Kühlwasser K reflektierte Licht.
Die Vorrichtung 2 ist parallel zu einer Hauptleitung 14 für das Kühlwasser K angeordnet. Über eine Nebenleitung 16 wird im offenen Zustand des Einlassventils 4 eine Teilmenge M an Kühlwasser K aus der Hauptleitung 14 in die Messzelle 12 gefördert. Wenn die Messzelle 12 insbesondere vollständig mit Kühlwasser K aufgefüllt ist, wird das Einlassventil 4 geschlossen. Anschließend wird eine Referenzmessung durchgeführt, bei der ein Referenzwert RW für die Trübung des Kühlwassers K gewonnen wird. Mit Trübung wird hierbei eine Veränderung der Transmissions- bzw. Reflektionsverhalten der Flüssigkeit aufgrund einer Bildung von Gasblasen in der Messzelle bezeichnet. Der Referenzwert RW wird in einer Analyse- und Steuereinheit 18, die ebenfalls Teil der Vorrichtung 2 ist, hinterlegt. Im gezeigten Ausführungsbeispiel ist lediglich eine Einheit zur Analyse der Messergebnisse und zur Steuerung der Komponenten der Vorrichtung 2 vorgesehen, alternativ können auch zwei separate Einheiten diese zwei Funktionen übernehmen.

Um den Einfluss von Verfärbungen oder Verschmutzungen im Kühlwasser K oder den Einfluss einer Alterung bzw. Schwächung der Lichtquelle 6 auf die Messung zu minimieren oder gar zu unterbinden, wird der Referenzwert RW in der Analyse- und Steuereinheit 18 mit einem vorgegebenen, fixen Grenzwert GW verglichen. Wenn der Referenzwerts RW dem Grenzwert GW entspricht oder diesen überschreitet, wird die Funktionalität der Vorrichtung 2 überprüft. Auf diese Weise erfolgt eine Selbstüberwachung der Vorrichtung 2, bei der Verschmutzungen oder Geräteausfälle frühzeitig erkannt werden. Im Normalfall ist der Referenzwert RW kleiner als der Grenzwert GW, dann ist das Kühlwasser K klar genug, um die Messung durchzuführen.

Nach der Referenzmessung wird der Druck pₘ in der Messzelle 12 herabgesetzt. Der Druck pₘ sowie die Temperatur Tₘ in der Messzelle 12 werden dabei mit Hilfe eines Drucksensors 20 und eines Temperatursensors 22 überwacht. Wenn das Kühlwasser K gelöstes Gas enthält, fällt dieses Gas bei der Entspannung des Kühlwassers K in der Messzelle 12 in Form von kleinen Blasen aus, die zu einer Trübung des Kühlwassers K führen. Da allerdings beim Erreichen des Siedepunkts (Siededruck und Siedetemperatur) das Kühlwasser K selbst in Form kleiner Blasen verdampft, die das Messergebnis für die Trübung beeinflussen, werden der Druck pₘ und gegebenenfalls die Temperatur Tₘ in der Messzelle 12 derart geregelt, dass sie stets oberhalb des Siedepunkts bleiben, insbesondere mindestens 50 mbar oberhalb des Siedepunkts bleiben.

Auf Grundlage der Lichtmessung bei Unterdruck wird ein Trübungswert TW für das Kühlwasser K in der Messzelle 12 ermittelt. Für die Ermittlung des Trübungswerts TW reicht es aus, wenn nur ein Lichtdetektor 8, 10 vorgesehen ist, der entweder das durchgelassene oder das reflektierte Licht detektiert. Für die Bestimmung des Trübungswerts TW wird insbesondere die Intensität des Lichts nach dem Kontakt mit dem Kühlwasser K in der Messzelle 12 herangezogen.

Der ermittelte Trübungswert TW wird der Analyse- und Steuereinheit 18 zugeführt und mit einem Schwellwert SW verglichen. Der Schwellwert SW kann als Zahlenwert vorgegeben sein, der Schwellwert SW kann jedoch alternativ Messung spezifisch definiert sein, indem er vom Referenzwert RW abhängig ist. Wenn der Trübungswert TW innerhalb eines vorgegebenen Toleranzbereichs bleibt und den Schwellwert SW nicht erreicht, lässt sich daraus schließen, dass im Kühlwasser K keine Gasblasen enthalten sind. In diesem Fall wird die Teilmenge M an Kühlwasser K, welche in der Messzelle 12 gemessen wurde, über eine Pumpe 24 zurück in die Hauptstromleitung 14 geführt. Die Pumpe 24 wird insbesondere von der Analyse- und Steuereinheit 18 angesteuert.

Wenn jedoch der Trübungswert TW den Schwellwert SW erreicht hat oder diesen überschreitet, ist dies ein Zeichen dafür, dass im Kühlwasser K Gas enthalten ist. Um Informationen über die Menge an Gas im Kühlwasser K zu erhalten, wird dabei berücksichtigt, bei welchem Druck pₘ und welcher Temperatur Tₘ die Trübung des Kühlwassers K in der Messzelle 12 auftritt. Die Anzeige 26 kann dabei dazu verwendet werden, den Gasanteil im Kühlwasser K anzuzeigen. Ergänzend dazu kann über die Anzeige 26 z.B. auch das Ergebnis des Vergleichs des Trübungswerts TW mit dem Schwellenwert SW dargestellt werden. Bei detektiertem Gas im Kühlwasser K, d.h. beim Eintreten einer Trübung in der Messzelle 12, wird außerdem ein Steuersignal 28 ausgegeben, welches veranlasst, dass eine Entgasungsvorrichtung, insbesondere ein hier nicht näher gezeigter, der Vorrichtung 2 nachgeschalteter Vakuum-Entgaser, aktiviert wird. Nach der Messung in der Messzelle 12 wird dabei auch die Teilmenge M an Kühlwasser K anschließend im Vakuum-Entgaser aufbereitet.

Die Vorrichtung 2 zur Überwachung des Gasgehalts im Kühlwasser K gemäß FIG 2 unterscheidet sich von der Vorrichtung 2 gemäß FIG 1 im Wesentlichen dadurch, dass die Anordnung im zweiten Ausführungsbeispiel für eine kontinuierliche Messung geeignet ist. Hierfür ist das Einlassventil 4 durch ein Regelventil 30 ersetzt. Das Regelventil 30 wird von der Analyse- und Steuereinheit 18 angesteuert, um den Zufluss von Kühlwasser K in die Messzelle 12 zu regulieren. Bei konstanter Drehzahl der Pumpe 24 wird das Regelventil 30 langsam geschlossen. Dabei sinkt der Druck pₘ in der Messzelle 12, bis sich Gasblasen bilden. Die Grenze für die Messung des Gasgehalts in der Teilmenge M liegt dabei bei ca. 50 mbar oberhalb des Siededrucks. Durch das Eintreten einer Trübung in der Messzelle 12 im Messbereich kann somit festgestellt werden, ob im Kühlwasser K Gas enthalten ist. Durch die weitere Auswertung des Drucks pₘ und der Temperatur Tₘ beim Eintreten der Trübung kann zudem der Gasgehalt gemessen werden.

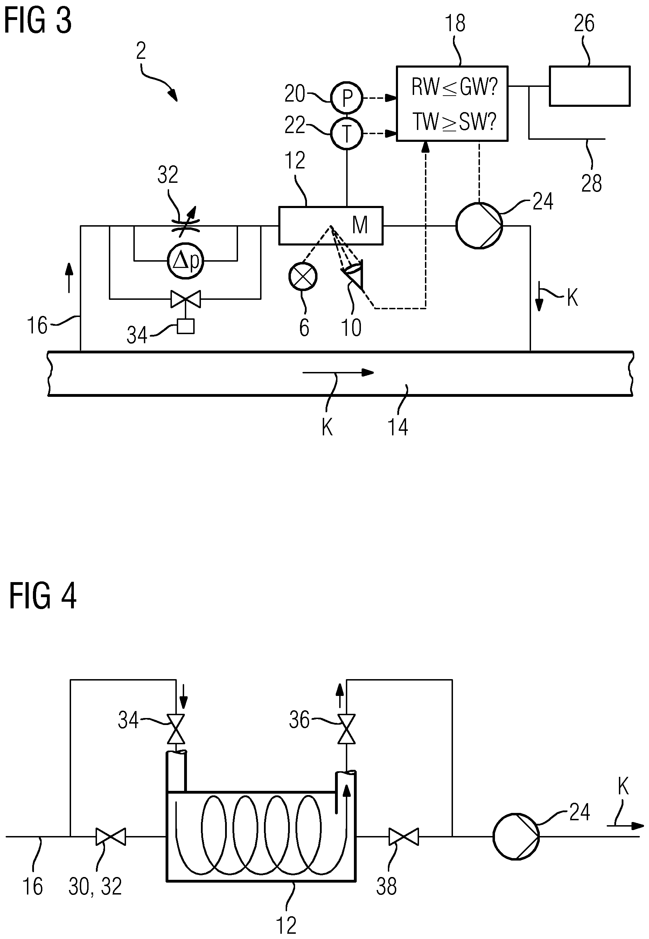

Gemäß FIG 3 strömt das Kühlwasser K durch ein fixes Drosselventil 32 in die Messzelle 12. Die Pumpendrehzahl wird so geändert, dass sich in der Messzelle 12 ein immer niedrigerer Druck einstellt, bis Trübung eintritt. Die Messung beginnt, wenn über dem Drosselventil 32 insbesondere ein Druckunterschied von mindestens 100 mbar entsteht.
In allen gezeigten Figuren erfolgt die Messung des Trübungswerts TW mit Hilfe einer Teilmenge M an Kühlwasser K, welche durch die Nebenleitung 16 umgeleitet wird. Alternativ wird die Vorrichtung 2 zur Überwachung des Gasgehalts im Kühlwasser K direkt in der Hauptleitung 14 integriert, so dass der gesamte Kühlmittelstrom durch die Vorrichtung 2 zu Messzwecken geleitet wird.

Die Messung wird in der Regel bei einem Differenzdruck von ca. 100 bar unter dem Anfangsdruck gestartet.

Darüber hinaus ist in allen gezeigten Ausführungen vor der Messzelle 12 ein Spülventil 34 mit großem Durchmesser vorgesehen, welches nur in FIG 3 gezeigt ist. Wenn sich Gasblasen an der Wand der Messzelle 12 festgesetzt haben, werden diese mit Hilfe des Spülventils 34, das einen großen Durchfluss zulässt, weggeschwemmt. Eine Verfälschung der Messergebnisse wird somit vermieden.

Die Spülung der Messzelle 12 wird anhand FIG 4 im Detail erläutert. Die Messzelle 12 wird ergänzt durch zwei Anschlüsse, die eine Strömung innerhalb der Messzelle 12 erzeugen, die sich schraubenförmig um die Mittelachse windet. Dies kann erreicht werden, wenn die Anschlüsse für den Spülzyklus radial angeordnet werden wie in FIG 4 gezeigt. Dazu sind außer dem bereits berücksichtigten Spülventil 34 zwei weitere Ventile 36, 38 erforderlich, die zwischen Spülung und Messung umschalten.

Für die Messung wird das Spülventil 34 bzw. 36 geschlossen und das Ventil 38 geöffnet. Dabei entsteht eine axiale Strömung durch die Messzelle 12.

Zur Reinigung der Wände von anhaftenden Blasen werden die Spülventile 34 und 36 geöffnet, Ventil 38 geschlossen und es entsteht eine rotierende Strömung. Durch die Rotation des Messzelleninhalts erreicht man an der Wand eine höhere Strömungsgeschwindigkeit, als wenn die Messzelle 12 bei gleichen Ventildurchmessern axial durchströmt würde. Blasen werden dadurch besser fortgeschwemmt. Für Spülventile 34, 36 können bei radialer Durchströmung dann kleinere Durchmesser verwendet werden, als bei rein axialer Durchströmung der Messzelle 12. Alternativ könnte das Spülventil 34 durch ein Drosselventil oder ein Regelventil ersetzt werden.

## Patentansprüche

1. Verfahren zur Überwachung und Messung eines Gasgehalts in einer Flüssigkeit (K), wobei
- zumindest eine Teilmenge (M) der Flüssigkeit (K) in eine Messzelle (12) gefördert wird,
- anschließend in der Messzelle (12) ein Unterdruck erzeugt wird,
- Druck (pₘ) und Temperatur (Tₘ) in der Messzelle (12) gemessen werden und
- der Gasgehalt in der Flüssigkeit (K) auf der Grundlage des Henry-Gesetzes in Abhängigkeit von dem gemessenen Druck und der Temperatur ermittelt wird,
**dadurch gekennzeichnet, dass**
- die Teilmenge (M) mit einem wellenförmigen Messsignal bestrahlt wird,
- das Messsignal nach dem Kontakt mit der Teilmenge (M) gemessen wird,
- die Trübung (TW) der Flüssigkeit (K) detektiert und mit einem Schwellwert (SW) verglichen wird, während der Unterdruck erzeugt wird, und
- der Gasgehalt in der Flüssigkeit (K) auf der Grundlage des Henry-Gesetzes ermittelt wird, wenn die Trübung (TW) den Schwellwert (SW) erreicht oder überschreitet, ohne dass der Siedepunkt der Flüssigkeit erreicht worden ist.

2. Verfahren nach Anspruch 1, **dadurch gekennzeichnet, dass** die Grenze für die Messung des Gasgehalts bei 50 mbar oberhalb des Siededrucks liegt.

3. Verfahren nach Anspruch 1 oder 2, **dadurch gekennzeichnet, dass** die Trübung optisch durch Lichtmessung detektiert wird.

4. Verfahren nach einem der vorangehenden Ansprüche , **dadurch gekennzeichnet, dass** die Trübung akustisch durch Ultraschallmessung detektiert wird.

5. Verfahren nach einem der vorhergehenden Ansprüche, **dadurch gekennzeichnet, dass** der Druck (pₘ) und/oder die Temperatur (Tₘ) in der Messzelle (12) derart angesteuert werden, dass sie oberhalb des Siedepunkts bleiben.

6. Verfahren nach einem der vorhergehenden Ansprüche, **dadurch gekennzeichnet, dass** bevor der Unterdruck erzeugt wird, ein Referenzwert (RW) für die Trübung detektiert wird und dass der Schwellwert (SW) auf Grundlage des Referenzwertes (RW) berechnet wird.

7. Verfahren nach Anspruch 6, **dadurch gekennzeichnet, dass** zur Funktionsüberwachung der Referenzwert (RW) mit einem Grenzwert (GW) verglichen wird.

8. Verfahren nach einem der vorhergehenden Ansprüche, **dadurch gekennzeichnet, dass** zur Spülung der Messzelle (12) der Durchfluss der Flüssigkeit (K) durch die Messzelle (12) erhöht wird, um Gasblasen von einer Wand der Messzelle (12) fortzuschwemmen.

9. Verfahren nach Anspruch 8, **dadurch gekennzeichnet, dass** von einer axialen Strömung durch die Messzelle (12) zur Messung auf eine rotierende Strömung zur Spülung umgeschaltet wird.

10. Vorrichtung (2) zur Überwachung und Messung eines Gasgehalts in einer Flüssigkeit (K), umfassend:
- eine Messzelle (12) zur Aufnahme zumindest einer Teilmenge (M) der Flüssigkeit (K),
- Mittel zum Füllen der Messzelle (12) mit zumindest einer Teilmenge (M) der Flüssigkeit (K) und zum anschließenden Erzeugen von Unterdruck in der Messzelle (12),
- Sensoren (20, 22) zum Bestimmen eines Druck (p) und einer Temperatur (T) in der Messzelle (12),
- sowie eine Analyse- und Steuereinheit (18), die dazu ausgebildet ist, den Gasgehalt in der Flüssigkeit (K) auf der Grundlage des Henry-Gesetzes in Abhängigkeit von dem gemessenen Druck und der Temperatur zu ermitteln,
**dadurch gekennzeichnet,**
- **dass** ein Lichtsender (6) und Lichtdetektor (8) zur Detektion der Trübung (TW) der Flüssigkeit (K) in der durchsichtigen Messzelle (12) vorhanden sind und
- **dass** die Analyse- und Steuereinheit (18) ferner dazu ausgebildet ist, die detektierte Trübung (TW) während der Erzeugung des Unterdrucks mit einem Schwellwert (SW) zu vergleichen und den Gasgehalt in der Flüssigkeit (K) auf der Grundlage des Henry-Gesetzes zu ermitteln, wenn die Trübung (TW) den Schwellwert (SW) erreicht oder überschreitet ohne dass der Siedepunkt der Flüssigkeit erreicht worden ist.

11. Verwendung einer Vorrichtung (2) nach Anspruch 10 zur Messung des Gasgehaltes in einem Kühlmittel (K) einer elektrochemischen Zelle, insbesondere einer Brennstoffzelle.

## Claims

1. Method for monitoring and measuring a gas content in a liquid (K), wherein
- at least one sub-quantity (M) of the liquid (K) is conveyed into a measurement cell (12),
- a vacuum is then created in the measurement cell (12),
- pressure (pₘ) and temperature (Tₘ) are measured in the measurement cell and
- the gas content in the liquid (K) is determined on the basis of Henry's law as a function of the measured pressure and temperature,
**characterised in that**
- the sub-quantity (M) is irradiated with a wavelike measurement signal,
- the measurement signal is measured after contact with the sub-quantity (M),
- the turbidity (TW) of the liquid (K) is detected and compared with a threshold value (SW), while the vacuum is being created, and
- the gas content in the liquid (K) is determined on the basis of Henry's law, if the turbidity (TW) reaches or exceeds the threshold value (SW) without the boiling point of the liquid having been reached.

2. Method according to claim 1, **characterised in that** the threshold for measuring the gas content lies at 50 mbar above the boiling pressure.

3. Method according to claim 1 or 2, **characterised in that** the turbidity is detected optically by light measurement.

4. Method according to one of the preceding claims, **characterised in that** the turbidity is detected acoustically by ultrasound measurement.

5. Method according to one of the preceding claims, **characterised in that** the pressure (pₘ) and/or the temperature (Tₘ) in the measurement cell (12) are controlled such that they remain above the boiling point.

6. Method according to one of the preceding claims, **characterised in that**, before the vacuum is created, a reference value (RW) for the turbidity is detected and that the threshold value (SW) is calculated on the basis of the reference value (RW).

7. Method according to claim 6, **characterised in that**, for functional monitoring, the reference value (RW) is compared with a limit value (GW).

8. Method according to one of the preceding claims, **characterised in that**, for flushing the measurement cell (12) the throughflow of the liquid (K) through the measurement cell (12) is increased, in order to flush away gas bubbles from a wall of the measurement cell (12).

9. Method according to claim 8, **characterised in that** a switch is made from an axial flow through the measurement cell (12) for measurement to a rotational flow for flushing.

10. Device (2) for monitoring and measuring a gas content in a liquid (K), comprising:
- A measurement cell (12) for receiving at least one sub-quantity (M) of the liquid (K),
- Means for filling the measurement cell (12) with at least one sub-quantity (M) of the liquid (K) and for subsequently creating a vacuum in the measurement cell (12),
- Sensors (20, 22) for determining a pressure (p) and a temperature (T) in the measurement cell (12),
- And also an analysis and control unit (18), which is configured to determine the gas content in the liquid (K) on the basis of Henry's law as a function of the measured pressure and the temperature,
**characterised in that**,
- a light transmitter (6) and light detector (8) are present for detection of the turbidity (TW) of the liquid (K) in the transparent measurement cell (12) and
- the analysis and control unit (18) is further configured to compare the detected turbidity (TW) during the creation of the vacuum with a threshold value (SW) and to determine the gas content in the liquid (K) on the basis of Henry's law, if the turbidity (TW) reaches or exceeds the threshold value (SW) without the boiling point of the liquid having been reached.

11. Use of a device (2) according to claim 10 for measurement of the gas content in a coolant (K) of an electrochemical cell, especially a fuel cell.

## Revendications

1. Procédé de contrôle et de mesure de la teneur en gaz d'un liquide (K) dans lequel
- on envoie au moins une quantité (M) partielle du liquide (K) dans une cellule (12) de mesure,
- on produit ensuite une dépression dans la cellule (12) de mesure,
- on mesure la pression (pₘ) et la température (Tₘ) dans la cellule (12) de mesure et
- on détermine la teneur en gaz du liquide (K) sur la base de la loi de Henry en fonction de la pression et de la température mesurées,
**caractérisé en ce que**
- on expose la quantité (M) partielle à un signal de mesure en forme d'ondes,
- on mesure le signal de mesure après le contact avec la quantité (M) partielle,
- on détecte la turbidité (TW) du liquide (K) et on la compare à une valeur (SW) de seuil pendant que l'on produit la dépression et
- on détermine la teneur en gaz du liquide (K) sur la base de la loi de Henry lorsque la turbidité (TW) atteint la valeur (SW) de seuil ou la dépasse, sans que le point d'ébullition du liquide ait été atteint.

2. Procédé suivant la revendication 1, **caractérisé en ce que** la limite pour la mesure de la teneur en gaz est à 50 mbar au dessus du point d'ébullition.

3. Procédé suivant la revendication 1 ou 2, **caractérisé en ce que** l'on détecte la turbidité optiquement par une mesure de lumière.

4. Procédé suivant l'une des revendications précédentes, **caractérisé en ce que** l'on détecte la turbidité acoustiquement par une mesure par ultrasons.

5. Procédé suivant l'une des revendications précédentes, **caractérisé en ce que** l'on règle la pression (pₘ) et/ou la température (Tₘ) dans la cellule (12) de mesure, de manière à ce qu'elle reste au dessus du point d'ébullition.

6. Procédé suivant l'une des revendications précédentes, **caractérisé en ce qu'**avant de produire la dépression, on détecte une valeur (RW) de référence de la turbidité et **en ce que** l'on calcule la valeur (SW) de seuil sur la base de la valeur (RW) de référence.

7. Procédé suivant la revendication 6, **caractérisé en ce que**, pour le contrôle du fonctionnement, on compare la valeur (RW) de référence à une valeur (GW) limite.

8. Procédé suivant l'une des revendications précédentes, **caractérisé en ce que**, pour laver la cellule (12) de mesure, on augmente le débit du liquide dans la cellule (12) de mesure, afin de débarrasser la paroi de la cellule (12) de mesure de bulles de gaz.

9. Procédé suivant la revendication 8, **caractérisé en ce que** l'on passe d'un écoulement axial dans la cellule (12) de mesure pour la mesure à un écoulement tournant pour le lavage.

10. Dispositif (2) de contrôle et de mesure de la teneur en gaz d'un liquide (K), comprenant :
- une cellule (12) de mesure de réception d'au moins une quantité (M) partielle du liquide (K),
- des moyens de remplissage de la cellule (12) de mesure d'au moins une quantité (M) partielle du liquide (K) et de production ensuite d'une dépression dans la cellule (12) de mesure,
- des capteurs (20, 22) de détermination d'une pression (p) d'une température (T) dans la cellule (12) de mesure,
- ainsi qu'une unité (18) d'analyse et de commande, qui est constituée pour déterminer la teneur en gaz du liquide (K) sur la base de la loi de Henry en fonction de la pression de la température mesurées,
**caractérisé**
- **en ce qu'**il y a un émetteur (6) de lumière et un détecteur (8) de lumière pour détecter la turbidité (TW) du liquide (K) dans la cellule (12) de mesure transparente et
- **en ce que** l'unité (18) d'analyse et de commande est constituée, en outre, pour comparer la turbidité (TW) détectée pendant la production de la dépression à une valeur (SW) de seuil et pour déterminer la teneur en gaz du liquide (K) sur la base de la loi de Henry lorsque la turbidité (TW) atteint la valeur (SW) de seuil ou la dépasse, sans que le point d'ébullition du liquide ait été atteint.

11. Utilisation d'un dispositif (2) suivant la revendication 10, pour mesurer la teneur en gaz d'un fluide (K) de refroidissement d'une cellule électrochimique, notamment d'une pile à combustible.
